# EUROPÄISCHE PATENTSCHRIFT

(11) **EP 3 387 983 B1**
(45) Veröffentlichungstag und Bekanntmachung des Hinweises auf die Patenterteilung: **07.10.2020**
(21) Anmeldenummer: 18165832.9
(22) Anmeldetag: 05.04.2018
(51) Int. Cl.: A61B 1/04, A61B 1/00, A61B 5/03, H01R 13/52, H05K 5/00, G02B 23/24, A61B 5/00

(54) **ENDOSKOP MIT DRUCKSENSOR**
ENDOSCOPE WITH PRESSURE SENSOR
ENDOSCOPE AVEC UN CAPTEUR DE PRESSION

(30) Priorität: 13.04.2017 DE 102017108029
(43) Veröffentlichungstag der Anmeldung: 17.10.2018
(73) Patentinhaber: Karl Storz SE & Co. KG, 78532 Baden-Württemberg Tuttlingen (DE)
(72) Erfinder: Ulmschneider, Daniel, 78532 Tuttlingen (DE)

(56) Entgegenhaltungen:
- EP-A1- 1 522 253
- DE-A1-102013 202 037
- DE-A1-102013 207 109
- US-A- 4 569 335

## Beschreibung

Die vorliegende Erfindung betrifft ein Endoskop mit einem Schaft, in dem ein Sensor zur Erfassung einer physikalischen Messgröße angeordnet ist.

Endoskope werden heute für vielerlei Anwendungen in Medizin und Technik verwendet. Ein Endoskop umfasst typischerweise einen starren, halbstarren oder flexiblen lang erstreckten Schaft, der zur Einführung in einen Hohlraum geeignet ist. Im distalen (d.h. beobachterfernen) Endbereich des Schafts ist zur Erzeugung eines Bildes eines Objektfelds in dem Hohlraum ein Endoskopobjektiv angeordnet, das mindestens eine Linse umfasst. Das endoskopische Bild kann über im Innern des Schafts angeordnete optische oder elektronische Mittel zum proximalen (beobachternahen) Ende des Endoskops weitergeleitet werden, wo es zur Betrachtung bzw. Anzeige für einen Benutzer zur Verfügung steht. Da in der Regel in dem beobachteten Hohlraum nicht ausreichend Licht vorhanden ist, ist ferner häufig innerhalb des Schafts ein Beleuchtungslichtleiter angeordnet, um Beleuchtungslicht an das distale Ende des Endoskops zu transportieren, wo es zur Beleuchtung des Hohlraums genutzt wird. Zur Einleitung des Beleuchtungslichts in das Endoskop ist oft in der Nähe des proximalen Endes ein Anschluss zum Anschließen eines Lichtleitkabels vorhanden, mit dem das Beleuchtungslicht von einer separaten Lichtquelle zugeführt werden kann.

Bei modernen Videoendoskopen sind am distalen Ende häufig ein oder mehrere Bildsensoren zur Aufnahme eines vom Objektiv erzeugten Bilds angeordnet. Alternativ können die Bildsensoren auch am proximalen Ende des Endoskops angeordnet sein, beispielsweise in einer Handhabe. Um eine Reinigung des Videoendoskops, beispielsweise in einem Autoklav zu ermöglichen, kann dieses fluiddicht, insbesondere gasdicht ausgebildet sein. Auf diese Weise werden die vorhandenen elektronischen Teile wie Bildsensoren, Kabel und Platinen vor eindringender Feuchtigkeit geschützt.

Bei der Durchführung einer endoskopischen Operation, wie auch zu diagnostischen Zwecken, kann es erforderlich sein, den Druck eines Fluids in der mit dem Endoskop beobachteten Körperhöhle zu messen. So kann beispielsweise ein Insufflationsgas oder eine Spülflüssigkeit in die Körperhöhle eingeleitet werden, um einen ausreichenden Arbeitsraum für endoskopische Manipulationen zu schaffen bzw. ein Operationsgebiet zu säubern und eine ungehinderte endoskopische Sicht zu ermöglichen. Für die sichere und schonende Durchführung der Insufflation bzw. Spülung ist die Messung des dabei in der Körperhöhle herrschenden Drucks vorteilhaft. Ebenso kann bei technischen Anwendungen die Erfassung eines Drucks in dem Hohlraum, in den der Schaft eingeführt ist, wünschenswert sein.

Aus der US4,569,335 ist ein Fiberskop bekannt, mit einem Schaft, in dem Beleuchtungslichtleiter und ein faseroptischer Drucksensor vorgesehen sind.

Die EP1522253A1 offenbart ein elektronisches Endoskop mit einem Schaft und einem proximalen Handgriff, wobei ein distaler Bildsensor vorgesehen ist, dessen Signale über elektrische Leitungen an eine im Handgriff befindliche Auswerteeinheit geleitet werden. Das Endoskop kann hermetisch abgedichtet sein, um es autoklavieren zu können.

Die DE102015000773A1 offenbart ein Endoskop mit einem Lichtleiterbündel, wobei ein faseroptischer Drucksensor in dem Lichtleiterbündel angeordnet ist.

Aus der DE102013207109A1 ist ein Videoendoskop mit einem distal in einem Schaft angeordneten Bildsensor bekannt, der über Kabel mit einer in einem Handgriff angeordneten Steuerelektronik verbunden ist. Durch das Instrument führt ein Instrumentenrohr. Das ganze Instrument ist nach außen und gegenüber dem Instrumentenrohr hermetisch abgedichtet.

Nachteilig an diesen aus dem Stand der Technik bekannten Instrumenten ist zum einen, dass das Signal eines Sensors zur Messung einer physikalischen Größe, wie einem Drucksensor, über eine separate Leitung aus dem Endoskop herausgeführt und einer externen Verarbeitungseinheit zugeführt werden muss. Zum anderen muss das ganze Endoskop abgedichtet werden, um es gut reinigen zu können, was einen erheblichen Aufwand bedeutet.

Es ist daher Aufgabe der vorliegenden Erfindung ein Endoskop vorzusehen, mit einem Sensor zur Erfassung einer physikalischen Messgröße, wobei das Endoskop eine Auswertung des Sensorsignals vornehmen kann und gleichzeitig gut reinigbar ist.

Diese Aufgabe wird durch eine Vorrichtung nach Anspruch 1 gelöst.

Vorteilhafte Weiterbildungen der Erfindung ergeben sich aus den Unteransprüchen.

Ein Endoskop umfasst einen langerstreckten Schaft, der starr, flexibel oder semiflexibel ausgebildet sein kann. Der Schaft ist zur Einführung in einen Hohlraum, beispielsweise in einen körperinneren Hohlraum eines menschlichen oder tierischen Körpers oder in einen Hohlraum eines technischen Gegenstands, ausgebildet und weist insbesondere eine hierfür geeignete Länge und einen entsprechenden Durchmesser auf. Das Endoskop ist insbesondere ein Videoendoskop mit einem oder mehreren Bildsensoren, die in einem distalen oder proximalen Bereich des Endoskops angeordnet sein können. Um die empfindlichen Bauteile des Endoskops bei der Anwendung und bei der Reinigung vor eindringender Feuchtigkeit zu schützen, ist in dem Endoskop ein fluiddichtes, insbesondere gasdichtes Gehäuse vorgesehen. Das Gehäuse kann die Bildsensoren, Leitungen oder auch Platinen mit weiteren elektronischen Bauteilen umgeben.

Um eine physikalische Größe erfassen zu können, weist das Endoskop einen entsprechenden Sensor auf. Dieser Sensor ist ein Drucksensor. Das Endoskop kann auch mehrere Sensoren aufweisen. Der Sensor kann elektronische Halbleiterbausteine umfassen. Der Drucksensor kann als faseroptischer Drucksensor ausgebildet sein. Um eine physikalische Größe beispielsweise der Endoskopumgebung sicher erfassen zu können, befindet sich der Sensor außerhalb des fluiddichten Gehäuses. Auf diese Weise wirken physikalische Bedingungen, die messtech nisch erfasst werden sollen, auf den Sensor ein. Von dem Sensor erstreckt sich eine Signalleitung, die die von dem Sensor erzeugten Messsignale weiterleitet. Dies können elektrische Signale sein mit einer entsprechend ausgebildeten Signalleitung, oder es kann sich um optische Signale handeln, die über eine optische Leitung wie eine Glasfaser geleitet werden. Die Signalleitung kann daher zumindest abschnittsweise zur Übertragung elektrischer Signale ausgebildet sein. Falls der Sensor ein faseroptischer Drucksensor ist, ist die Signalleitung zumindest abschnittsweise als optische Faser ausgebildet. Die Signalleitung kann auch in einem Abschnitt eine optische Leitung und einem anderen Abschnitt eine elektrische Leitung sein, wenn beispielsweise zwischen den Abschnitten ein optisches Signal in ein elektrisches umgewandelt wird oder umgekehrt.

Um nun bereits im Endoskop selbst eine Auswertung der von dem Sensor bereitgestellten Signale vornehmen zu können, weist das Endoskop eine entsprechende elektronische Auswerteeinheit auf. Die Auswerteeinheit ist, gegebenenfalls zusammen mit anderen elektronischen Bauteilen, in einem Innenraum des fluiddichten Gehäuses angeordnet. Die Signalleitung erstreckt sich von dem Sensor zu einer Koppelstelle an dem Gehäuse und ist weiter mit der Auswerteeinheit verbunden.

Das Endoskop kann weiter eine am proximalen Ende des Schafts angeordnete Handhabe aufweisen. Die Handhabe dient beispielsweise zum Ergreifen und Bedienen des Endoskops durch einen Anwender. Sie kann Bedienknöpfe, Hebel oder Ventile zur Steuerung verschiedener Funktionen des Endoskops aufweisen. Das fluiddichte Gehäuse kann insbesondere wenigstens teilweise innerhalb der Handhabe angeordnet sein. Häufig erzeugen elektronische Bauteile innerhalb des Gehäuses Wärme, die dann über die Handhabe abgeführt werden kann. Eine Abgabe von Wärme an einem distalen Ende des Endoskops ist meist nicht gewünscht, um einen beobachteten Hohlraum nicht zu erwärmen und damit möglicherweise zu beschädigen.

Die Koppelstelle ist bevorzugterweise im Bereich der Handhabe an dem Gehäuse angeordnet. Dort ist mehr Platz als im Schaft vorhanden, so dass an dieser Stelle die Signalleitung in den Innenraum des Gehäuses geführt werden kann.

Der Schaft des Endoskops weist eine Außenwand auf, wobei die Signalleitung zumindest abschnittsweise zwischen der Außenwand und dem fluiddicht ausgebildeten Gehäuse verläuft. Die Signalleitung verläuft insbesondere durch den Schaft bis in den Bereich der proximalen Handhabe und zur Koppelstelle.

Zwischen der Außenwand und dem fluiddichten Gehäuse können zudem Fasern zum Transport von Licht angeordnet sein. Diese werden beispielsweise durch eine externe oder im Handgriff befindliche Lichtquelle gespeist und transportieren das Licht zum distalen Ende des Endoskops um dort den Hohlraum zu beleuchten. Dabei können der Sensor und/oder die Signalleitung zumindest abschnittsweise zwischen den Fasern eingebettet sein. Hierzu kann beispielsweise ein Kanal zwischen den Lichtleitfasern vorgesehen sein, in dem Sensor und/oder Signalleitung angeordnet sind. Sensor und Signalleitung können auch mit den Lichtleitfasern verklebt sein. Diese Anordnung hat den Vorteil, dass der Sensor oder die Signalleitung platzsparend im Endoskopschaft untergebracht werden können, ohne den Durchmesser des Endoskops zu vergrößern, da der ohnehin für die Beleuchtungsfasern vorgesehene Raum mit genutzt wird.

An der Koppelstelle wird die Signalleitung in den Innenraum des fluiddichten Gehäuses geführt. Die Signalleitung kann ohne Unterbrechung durch die Koppelstelle durchgeführt und im Innenraum mit der Auswerteeinheit verbunden sein. Alternativ kann ein Abschnitt der Signalleitung auch an der Koppelstelle enden und ein weiterer Abschnitt die Koppelstelle im Innenraum mit der Auswerteeinheit verbinden. Die Auswerteeinheit kann auch unmittelbar hinter der Koppelstelle im Innenraum des Gehäuses angeordnet sein, so dass eine direkte Verbindung mit der Signalleitung im Bereich der Koppelstelle erfolgt. Die Abschnitte der Leitung können jeweils elektrische oder optische Leiter sein.

Die Koppelstelle kann, an der dem Innenraum zugewandten oder der gegenüberliegenden Seite, oder an beiden Seiten, einen Stecker oder eine Aufnahme zur Verbindung mit der Signalleitung und zur Übertragung elektrischer oder optischer Signale aufweisen. Die Signalleitung kann dazu mit komplementären Steckern oder Aufnahmen versehen sein. Stecker und Aufnahmen können in bekannter Weise leitende Kontakte zur Übertragung elektrischer Signale aufweisen. Alternativ kann es sich um Faserstecker- bzw. aufnahmen wie LWL-Steckverbinder handeln, die Lichtleiter auf optischem Wege verbinden können. Auf diese Weise kann eine einfache, zuverlässige Verbindung der Signalleitung oder der Abschnitte der Signalleitung mit der Koppelstelle gewährleistet werden, die zudem die Herstellung vereinfacht, da die Leitungen nur angesteckt werden müssen.

Die Koppelstelle ist fluiddicht, insbesondere gasdicht, ausgebildet. Damit wird sichergestellt, dass durch Reinigung oder während der Anwendung im Bereich der Koppelstelle keine Feuchtigkeit in den Innenraum des Gehäuses eindringen kann. Die Koppelstelle kann hierfür eine Abdichtung aufweisen. Beispielsweise kann eine durchgeführte Signalleitung im Bereich der Koppelstelle mit Klebstoff, wie einem Kunstharz, mit dem Gehäuse verbunden werden. Alternativ kann eine flexible Kunststoffdichtung vorgesehen sein, durch die die Signalleitung geführt wird. Falls Stecker oder Aufnahmen vorgesehen sind, können diese aus undurchlässigem Material dicht ausgebildet und abdichtend mit dem Gehäuse verbunden sein. Hierzu ist bekannt, elektrische Kontakte in ein abdichtendes Material einzubetten, beispielsweise in Form eines Glasvergusssteckers.

Die in dem fluiddichten Gehäuse angeordnete Auswerteeinheit erhält Signale von dem Sensor und wertet diese aus. Es kann sich dabei um elektrische Signale handeln, die beispielsweise durch eine Spannungs- oder Widerstandsänderung im Sensor erzeugt werden. Die Auswerteeinheit weist zum Zwecke der Auswertung passende elektrische Schaltungen auf, die das elektrische Messsignal verarbeiten. Die Signale können wie benötigt verstärkt oder umgewandelt werden. Zusätzlich kann die Auswerteeinheit elektronische Bauteile für eine weitergehende Signalverarbeitung aufweisen. Die Signale können korrigiert und dafür untereinander oder mit anderen Messsignalen abgeglichen werden. Beispielsweise können die Messungen eines Drucksensors, die temperaturabhängig sein können, durch die Messungen eines Temperatursensors korrigiert oder Messwerte rechnerisch angepasst werden. Die Auswerteeinheit kann einen Prozessor, Arbeitsspeicher oder Datenspeicher aufweisen. Messsignale können über einen gewissen Zeitraum von der Auswerteeinheit gespeichert und dann weiterverarbeitet oder weitergesendet werden. Auch kann die Auswerteeinheit dazu ausgebildet sein, einen Sensor, der Signale übermittelt, zu identifizieren um die Signale unterschiedlicher Sensoren voneinander unterscheiden zu können. Im Gehäuse können bei Bedarf Mittel zur Stromversorgung des Sensors vorgesehen sein.

Falls es sich um einen faseroptischen Sensor handelt, dessen Signale über die Signalleitung an die Auswerteeinheit übermittelt werden, kann die Auswerteeinheit Mittel zur Erfassung und Auswertung der optischen Signale aufweisen. Dies können Spektrometer, Interferometer, optische Gitter oder Lichtsensoren und opto-elektrische Wandler sein. Die Auswerteeinheit kann eine Lichtquelle zur Speisung des faseroptischen Sensors aufweisen. Auch in diesem Fall können wie zuvor zu den elektrischen Signalen ausgeführt elektronische Bauteile vorhanden sein. Des Weiteren können die Signale weiterer Sensoren in der Auswerteeinheit mit verarbeitet und die Ergebnisse miteinander verglichen und ausgewertet werden.

Die Auswertung der Messsignale in einer Auswerteeinheit im Endoskop selbst hat den Vorteil, dass die Signalleitung selbst nicht aus dem Instrument herausgeführt werden muss. Auch kann auf eine zusätzliche externe Auswerteeinheit verzichtet werden, was die Anordnung besonders kompakt macht.

Weiter kann das Endoskop eine Leitung aufweisen, die mit der Auswerteeinheit in Verbindung steht und geeignet ist, Signale oder Daten mit dem Ergebnis der Auswertung von der Auswerteeinheit zu einem außerhalb des Endoskops angeordneten Monitor oder medizinischen Gerät zu leiten. Häufig weisen Endoskope, insbesondere Videoendoskope, bereits eine Leitung auf, die das Videoendoskop mit Strom versorgt und Signale und Daten beispielsweise von einem Bildsensor von dem Endoskop zu einem externen Gerät zur Weiterverarbeitung, oder gleich zu einem Monitor zur Anzeige der Daten leitet. Idealerweise kann eine bereits vorhandene Leitung genutzt werden, um auch die Signale oder Daten von der Auswerteeinheit des Sensors nach außen zu übermitteln. Beispielsweise kann ein bereits ausgewertetes Messergebnis direkt auf einem Monitor angezeigt oder zur weiteren Berücksichtigung an ein medizinisches Gerät übermittelt werden, beispielsweise zur Druckregelung einer Insufflations- oder Flüssigkeitspumpe. Es ist dann keine weitere Leitung notwendig, die die Handhabung des Videoendoskops erschweren würde. Besonders einfach ist dies zu realisieren, wenn sich die Auswerteeinheit und ein Bildsensor, und/oder die Mittel zur Verarbeitung eines Bildsignals zusammen im Gehäuse im Bereich der Handhabe befinden. Sie können auf der gleichen Leiterplatte angeordnet sein und die Signale und Daten der Auswerteeinheit und der Bildverarbeitung können zusammen durch die vorhandene Leitung nach außen geleitet werden.

Alternativ oder zusätzlich weist das Endoskop einen Sender auf, der mit der Auswerteeinheit in Verbindung steht und geeignet ist, Signale oder Daten mit dem Ergebnis der Auswertung von der Auswerteeinheit drahtlos zu einem außerhalb des Endoskops angeordneten Monitor oder medizinischen Gerät zu senden. Dies kann in an sich bekannter Weise über elektromagnetische Wellen geschehen (WLAN, Bluetooth oder andere bekannte Verfahren). Ein externes Gerät oder ein Monitor weisen eine Empfangseinheit auf, um die Signale zu empfangen und ein Messergebnis direkt anzuzeigen oder weiter zu verarbeiten.

Weitere Aspekte der Erfindung ergeben sich aus der nachfolgenden Beschreibung der bevorzugten Ausführungsbeispiele und den beigefügten Zeichnungen. Es zeigen:
- Figur 1: ein Ausführungsbeispiel eines erfindungsgemäßen Endoskops im Längsschnitt
- Figur 2: ein Ausführungsbeispiel einer Koppelstelle
- Figur 3: ein weiteres Ausführungsbeispiel einer Koppelstelle
- Figur 4: ein medizinisches System mit einem Endoskop

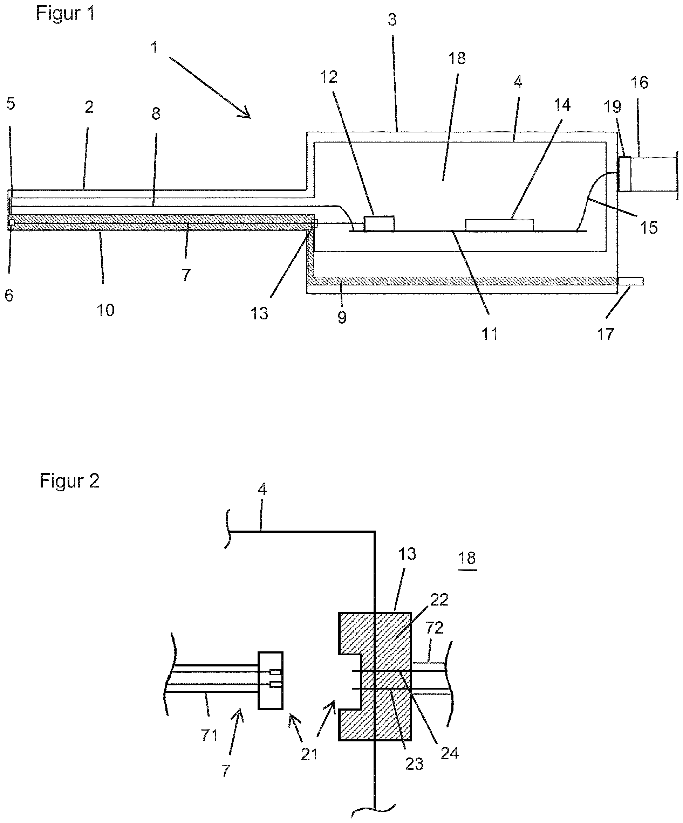

Das in Figur 1 dargestellte erfindungsgemäße Endoskop 1 ist ein Videoendoskop und weist einen Schaft 2 und eine Handhabe 3 auf, die sich proximal an den Schaft anschließt. Im Inneren der Handhabe 3 und des Schafts 2 erstreckt sich ein autoklavdichtes (d.h. fluiddichtes, insbesondere gasdichtes) Gehäuse 4. Am distalen Ende des Gehäuses 4 befindet sich ein Bildaufnehmer 5 bestehend aus Bildsensor und davor angeordnetem Objektiv. Licht gelangt von distal durch ein Fenster in den Schaft 2 und das Gehäuse 4 und wird vom Objektiv auf den Bildsensor abgebildet. Eine Datenleitung 8 leitet die vom Bildsensor erzeugten Daten weiter nach proximal zu einer in dem Gehäuse 4 gelegenen Platine 11. Auf der Platine ist eine Bildverarbeitungseinheit 14 montiert, die die vom Bildsensor empfangenen Daten für die Weiterleitung aus dem Endoskop 1 heraus vorbereitet. Das Gehäuse 4 ist rundherum abgedichtet, so dass bei einer Beaufschlagung des Endoskops 1 mit hohem atmosphärischem Druck und mit Feuchtigkeit, wie dies bei der Reinigung in einem Autoklav der Fall ist, keine Feuchtigkeit in das Gehäuse 4 eindringt. Zu diesem Zweck kann das distale Fenster entsprechend gegenüber dem Schaft 2 abgedichtet, z.B. eingelötet sein. Das Gehäuse 4 schützt auf diese Weise die elektronischen Komponenten auf der Platine 11, den Bildaufnehmer 5 und die Leitung 8 innerhalb des Gehäuses 4. Außerhalb des Gehäuses 4 befindet sich distal im Schaft ein Drucksensor 6, dessen druckempfindliche Fläche mit der Umgebung des Endoskops 1 in Verbindung steht, so dass ein Umgebungsdruck gemessen werden kann. Von dem Drucksensor 6 erstreckt sich eine Signalleitung 7 durch den Schaft 2 zu einer Koppelstelle 13 am Gehäuse 4. Die Koppelstelle 13 befindet sich in diesem Ausführungsbeispiel im Bereich des distalen Endes der Handhabe 3 am fluiddicht ausgebildeten Gehäuse 4. Die Signalleitung 7 verläuft dabei zwischen einer Außenwand 10 des Schafts 2 und dem Gehäuse 4. Um den Drucksensor 6 und die Signalleitung 7 herum sind Lichtleitfasern 9 angeordnet und umgeben diese. Diese erstrecken sich bis zu einem proximalen Lichtanschluss 17, an den ein Lichtleitkabel zur Verbindung mit einer externen Lichtquelle angeschlossen werden kann (nicht dargestellt).

An der Koppelstelle 13 wird die Signalleitung 7 in den Innenraum 18 des Gehäuses 4 geleitet. Die Koppelstelle 13 ist autoklavdicht ausgebildet, d.h. es kann wie zuvor erläutert beim Autoklavieren des Endoskops 1 kein Dampf in das Gehäuse 4 eindringen. Die Signalleitung 7 verläuft im Gehäuse 4 zu einer auf der Platine 11 montierten Auswerteeinheit 12. Die Signale des Drucksensors 6 werden somit über die Signalleitung 7 durch den Schaft 2 in das Gehäuse 4 und zur Auswerteeinheit 12 geleitet. Die Auswerteeinheit 12 weist eine Reihe von Schaltungen, einen Prozessor, einen Arbeitsspeicher und einen Datenspeicher auf (nicht dargestellt). Sie wertet die vom Sensor 6 erhaltenen elektrischen oder optischen Signale aus und bereitet sie für die Anzeige an einem externen Monitor 42 vor.

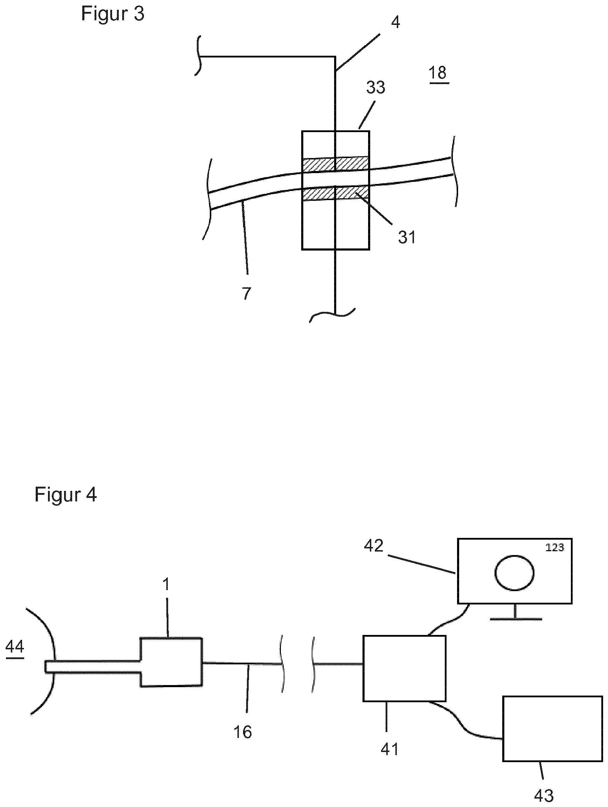

Die Auswerteeinheit 12 und die Bildverarbeitungseinheit 14 befinden sich auf derselben Platine 11. Diese ist über eine Verbindung 15 und eine nicht dargestellte Schnittstelle mit einem außerhalb des Endoskops 1 angebrachten Videokabel 16 verbunden. Das Videokabel 16 verbindet das Endoskop 1 mit einem Kontrolleinheit 41 und im weiteren Verlauf mit dem externen Monitor 42, wie dies in Figur 4 genauer dargestellt ist. Über das Videokabel 16 werden also sowohl die von der Auswerteeinheit 12 erhaltenen Ergebnisse der Auswertung des Sensorsignals, als auch die Daten von der Bildverarbeitungseinheit 14 von dem Endoskop 1 zu einem Monitor geleitet. Auf diese Weise wird die Zahl der am Endoskop 1 angeschlossenen Kabel auf ein Minimum reduziert. Alternativ kann der Monitor 42 auch statt des Videokabels 16 direkt am proximalen Ende des Endoskops 1 angebracht sein und die Daten von der Auswerteeinheit 12 und der Bildverarbeitungseinheit 14 empfangen. Im vorliegenden Beispiel weist das Endoskop 1 an der Schnittstelle zum Videokabel 16 außerdem zusätzlich einen Sender 19 auf. Dieser ist dazu ausgebildet die von der Auswerteeinheit 12 und der Bildverarbeitungseinheit 14 bereitgestellten Signale und Daten drahtlos an eine externe Empfangseinheit zu senden, die beispielsweise zu einem Monitor oder einem medizinischen Gerät gehört. Auf diese Weise kann bei Bedarf das Kabel 16 ganz weggelassen werden, was die Handhabung des Endoskops 1 verbessert. Das Endoskop 1 weist dann eine eigene Energiequelle auf (nicht dargestellt).

Figur 2 zeigt eine mögliche Ausgestaltung einer Koppelstelle 13, wie sie in einem Endoskop 1 gemäß Figur 1 verwendet werden kann.

Die Koppelstelle 13 befindet sich an einem Abschnitt des Gehäuses 4 innerhalb des Endoskops 1. Sie ist als Steckverbindung 21 ausgebildet und weist an der Außenseite des Gehäuses 4 eine Aufnahme mit zwei Kontakten 23 und 24 auf. Ein erster Abschnitt 71 der Signalleitung 7, die hier als elektrische Signalleitung ausgebildet ist, weist an seinem Ende einen Stecker auf, der komplementär zur Aufnahme an der Koppelstelle 13 ausgebildet ist. Die Kontakte 23 und 24 sind in eine Dichtung 22 eingebettet und erstrecken sich durch die Dichtung 22 und die Gehäusewand 4 zu einer im Innenraum 18 des Gehäuses 4 gelegenen Seite der Koppelstelle 13. Die Koppelstelle 13 ist insbesondere als Glasvergussstecker ausgebildet. Dieser ist abdichtend mit dem Gehäuse 4 verbunden. Im Innenraum 18 sind die Kontakte 23 und 24 innerhalb eines zweiten Abschnitts 72 der Signalleitung 7 weitergeführt, die fest mit der Koppelstelle 13 verbunden ist. Der zweite Abschnitt 72 ist im weiteren Verlauf mit der Auswerteeinheit 12 verbunden und überträgt die vom Drucksensor 6 über den ersten Abschnitt 71 der Signalleitung 7 und die Koppelstelle 13 übermittelten Signale an die Auswerteeinheit 12. Die Steckverbindung kann auch mehrere Kontakte aufweisen, je nachdem, wie viele Kontakte für die Signalübertragung benötigt werden. In Figur 3 ist eine alternative Ausgestaltung einer Koppelstelle 33, wie sie in einem Endoskop 1 gemäß Figur 1 verwendet werden kann.

Die Koppelstelle 33 befindet sich wie zuvor an einem Abschnitt des Gehäuses 4 innerhalb des Endoskop 1. Sie ist als Durchführung ausgebildet, die sich von außerhalb des Gehäuses 4 durch die Koppelstelle 33 zum Innenraum 18 erstreckt. Die Signalleitung 7 ist ohne Unterbrechung durch die Koppelstelle 33 geführt und kontaktiert im Innenraum 18 wie zuvor ausgeführt die Auswerteeinheit 12. Um zu garantieren, dass die Koppelstelle 33 und damit das Gehäuse 4 gasdicht ausgebildet ist, ist die Signalleitung 7 im Bereich der Koppelstelle 33 in diese eingeklebt, also von einem Klebstoff 31 umgeben. Die Signalleitung 7 ist hier beispielsweise ein Lichtleitkabel, das sich von einem faseroptischen Drucksensor 6 zur Auswerteeinheit 12 erstreckt.

Figur 4 zeigt ein System, in dem ein erfindungsgemäßes Endoskop angeordnet sein kann. Über ein Videokabel 16 ist das Endoskop 1 mit einer CCU (Kamerakontrolleinheit, engl. Camera control unit, kurz CCU) 41 verbunden. Diese empfängt die Videodaten vom Endoskop 1 und verarbeitet diese für die Darstellung des Videos auf dem angeschlossenen Monitor 42. Ebenfalls über das Videokabel 16 werden die Daten des Sensors 6 und der Auswerteeinheit 12 an die CCU 41 und weiter an den Monitor 42 übertragen. Im vorliegenden Beispiel eines Drucksensors 6 wird ein in einer Körperhöhle 44 gemessener Druck am Monitor 42 angezeigt. Außerdem ist mit der CCU 41 auch eine Insufflationspumpe 43 verbunden. Diese empfängt ebenfalls die Daten von Sensor 6 bzw. der Auswerteeinheit 12. Mit Hilfe der so erhaltenen Information über den aktuellen Druck in der Körperhöhle 44 kann die Insufflationspumpe 43 gesteuert und der Druck nach Bedarf angepasst werden.

### Bezugszeichenliste:

- 1: Endoskop
- 2: Schaft
- 3: Handhabe
- 4: Gehäuse
- 5: Bildaufnehmer
- 6: Drucksensor
- 7: Signalleitung
- 8: Datenleitung
- 9: Lichtleitfasern
- 10: Außenwand
- 11: Platine
- 12: Auswerteeinheit
- 13: Koppelstelle
- 14: Bildverarbeitungseinheit
- 15: Verbindung
- 16: Videokabel mit Stecker
- 17: Lichtanschluss
- 18: Innenraum
- 19: Sender
- 21: Steckverbindung
- 22: Dichtung
- 23, 24: Kontakte
- 31: Klebstoff
- 33: Koppelstelle
- 41: CCU
- 42: Monitor
- 43: medizinisches Gerät
- 71: erster Abschnitt
- 72: zweiter Abschnitt

## Patentansprüche

1. Endoskop (1) mit einem langerstreckten Schaft (2),
einem in dem Endoskop (1) angeordneten, fluiddicht ausgebildeten Gehäuse (4), einem in dem Schaft (2) angeordneten Sensor (6) zur Erfassung einer physikalischen Messgröße,
wobei sich der Sensor (6) außerhalb des fluiddichten Gehäuses (4) befindet,
und mit einer Signalleitung (7), die sich von dem Sensor (6) zu einer Koppelstelle (13, 33) an dem fluiddichten Gehäuse (4) erstreckt,
**dadurch gekennzeichnet, dass** die Koppelstelle (13, 33) die Signalleitung (7) mit einem Innenraum (18) des fluiddichten Gehäuses (4) verbindet und die Signalleitung (7) mit einer in dem Innenraum (18) angeordneten Auswerteeinheit (12) verbunden ist, wobei der Sensor (6) ein Drucksensor ist.

2. Endoskop (1) nach Anspruch 1, **dadurch gekennzeichnet, dass** der Sensor (6) ein faseroptischer Drucksensor ist, wobei die Signalleitung (7) zumindest abschnittsweise als optische Faser ausgebildet ist.

3. Endoskop (1) nach Anspruch 1 bis 2, wobei die Signalleitung (7) zumindest abschnittsweise zur Übertragung elektrischer Signale ausgebildet ist.

4. Endoskop (1) nach einem der vorhergehenden Ansprüche, wobei das Endoskop (1) eine am proximalen Ende des Schafts (2) angeordnete Handhabe (3) aufweist und das fluiddichte Gehäuse (4) wenigstens teilweise innerhalb der Handhabe (3) angeordnet ist.

5. Endoskop (1) nach einem der vorhergehenden Ansprüche, wobei die Koppelstelle (13, 33) im Bereich der Handhabe (3) an dem Gehäuse (4) angeordnet ist.

6. Endoskop (1) nach einem der vorhergehenden Ansprüche, wobei der Schaft (2) eine Außenwand (10) aufweist und die Signalleitung (7) zumindest abschnittsweise zwischen der Außenwand (10) und dem fluiddicht ausgebildeten Gehäuse (4) verläuft.

7. Endoskop (1) nach einem der vorhergehenden Ansprüche, wobei der Schaft (2) eine Außenwand (10) aufweist und zwischen Außenwand (10) und fluiddichtem Gehäuse (4) Fasern (9) zum Transport von Licht angeordnet sind, **dadurch gekennzeichnet, dass** der Sensor (6) und/oder die Signalleitung (7) zumindest abschnittsweise zwischen den Fasern (9) eingebettet ist.

8. Endoskop (1) nach einem der vorhergehenden Ansprüche, **dadurch gekennzeichnet, dass** die Koppelstelle (13) zur Übertragung elektrischer oder optischer Signale einen Stecker oder eine Aufnahme zur Verbindung mit der Signalleitung (7) aufweist.

9. Endoskop (1) nach einem der vorhergehenden Ansprüche, **dadurch gekennzeichnet, dass** die Koppelstelle (13, 33) fluiddicht ausgebildet ist.

10. Endoskop (1) nach einem der vorhergehenden Ansprüche, **dadurch gekennzeichnet, dass** die Auswerteeinheit (12) die von dem Sensor (6) erhaltenen Signale auswertet.

11. Endoskop (1) nach Anspruch 10, **dadurch gekennzeichnet, dass** das Endoskop (1) eine Leitung (15, 16) aufweist, die mit der Auswerteeinheit (12) in Verbindung steht und geeignet ist, Signale oder Daten mit dem Ergebnis der Auswertung von der Auswerteeinheit (12) zu einem außerhalb des Endoskops (1) angeordneten Monitor (42) oder medizinischen Gerät (43) zu leiten.

12. Endoskop (1) nach Anspruch 10 oder 11, **dadurch gekennzeichnet, dass** das Endoskop (1) einen Sender (19) aufweist, der mit der Auswerteeinheit (12) in Verbindung steht und geeignet ist, Signale oder Daten mit dem Ergebnis der Auswertung von der Auswerteeinheit (12) drahtlos zu einem außerhalb des Endoskops (1) angeordneten Monitor (42) oder medizinischen Gerät (43) zu senden.

## Claims

1. Endoscope (1) having an elongate shaft (2),
a housing (4), which is arranged in the endoscope (1) and which has a fluid-tight embodiment,
a sensor (6), arranged in the shaft (2), for capturing a physical measurement variable,
the sensor (6) being situated outside of the fluid-tight housing (4),
and having a signal line (7), which extends from the sensor (6) to a coupling point (13, 33) at the fluid-tight housing (4),
**characterized in that** the coupling point (13, 33) connects the signal line (7) to an interior (18) of the fluid-tight housing (4) and the signal line (7) is connected to an evaluation unit (12) arranged in the interior (18), the sensor (6) being a pressure sensor.

2. Endoscope (1) according to Claim 1, **characterized in that** the sensor (6) is a fibre-optic pressure sensor, the signal line (7) being embodied as an optical fibre, at least in sections.

3. Endoscope (1) according to Claim 1 to 2, wherein the signal line (7) is embodied to transmit electrical signals, at least in sections.

4. Endoscope (1) according to any one of the preceding claims, wherein the endoscope (1) has a handle (3) which is arranged at the proximal end of the shaft (2) and the fluid-tight housing (4) is at least partly arranged within the handle (3).

5. Endoscope (1) according to any one of the preceding claims, wherein the coupling point (13, 33) is arranged on the housing (4) in the region of the handle (3).

6. Endoscope (1) according to any one of the preceding claims, wherein the shaft (2) has an external wall (10) and the signal line (7) extends, at least in sections, between the external wall (10) and the housing (4) with the fluid-tight embodiment.

7. Endoscope (1) according to any one of the preceding claims, wherein the shaft (2) has an external wall (10) and fibres (9) for transporting light are arranged between the external wall (10) and the fluid-tight housing (4), **characterized in that** the sensor (6) and/or the signal line (7) are embedded, at least in sections, between the fibres (9).

8. Endoscope (1) according to any one of the preceding claims, **characterized in that** the coupling point (13) has a plug or a receptacle, to be connected to the signal line (7), for the purposes of transmitting electrical or optical signals.

9. Endoscope (1) according to any one of the preceding claims, **characterized in that** the coupling point (13, 33) has a fluid-tight embodiment.

10. Endoscope (1) according to any one of the preceding claims, **characterized in that** the evaluation unit (12) evaluates the signals received from the sensor (6) .

11. Endoscope (1) according to Claim 10, **characterized in that** the endoscope (1) has a line (15, 16), which is connected to the evaluation unit (12) and which is suitable for transmitting signals or data containing the results of the evaluation from the evaluation unit (12) to a monitor (42) or a medical device (43) arranged outside of the endoscope (1).

12. Endoscope (1) according to Claim 10 or 11, **characterized in that** the endoscope (1) has a transmitter (19), which is connected to the evaluation unit (12) and which is suitable for wirelessly transmitting signals or data containing the results of the evaluation from the evaluation unit (12) to a monitor (42) or a medical device (43) arranged outside of the endoscope (1).

## Revendications

1. Endoscope (1), comprenant une tige allongée (2),
un boîtier (4) réalisé de manière étanche aux fluides et disposé dans l'endoscope (1),
un capteur (6) disposé dans la tige (2) pour détecter un paramètre physique,
le capteur (6) se trouvant à l'extérieur du boîtier étanche aux fluides (4),
et comprenant une ligne de signal (7) qui s'étend du capteur (6) jusqu'à un point de couplage (13, 33) sur le boîtier étanche aux fluides (4),
**caractérisé en ce que** le point de couplage (13, 33) relie la ligne de signal (7) à un espace intérieur (18) du boîtier étanche aux fluides (4) et la ligne de signal (7) est reliée à une unité d'évaluation (12) disposée dans l'espace intérieur (18), le capteur (6) étant un capteur de pression.

2. Endoscope (1) selon la revendication 1, **caractérisé en ce que** le capteur (6) est un capteur de pression à fibre optique, la ligne de signal (7) étant réalisée au moins en partie comme une fibre optique.

3. Endoscope (1) selon les revendications 1 et 2, dans lequel la ligne de signal (7) est réalisée au moins en partie pour transmettre des signaux électriques.

4. Endoscope (1) selon l'une quelconque des revendications précédentes, dans lequel l'endoscope (1) présente une poignée (3) disposée à l'extrémité proximale de la tige (2), et le boîtier étanche aux fluides (4) est disposé au moins en partie à l'intérieur de la poignée (3).

5. Endoscope (1) selon l'une quelconque des revendications précédentes, dans lequel le point de couplage (13, 33) est disposé sur le boîtier (4) au niveau de la poignée (3).

6. Endoscope (1) selon l'une quelconque des revendications précédentes, dans lequel la tige (2) présente une paroi extérieure (10), et la ligne de signal (7) s'étend au moins par endroits entre la paroi extérieure (10) et le boîtier (4) réalisé de manière étanche aux fluides.

7. Endoscope (1) selon l'une quelconque des revendications précédentes, dans lequel la tige (2) présente une paroi extérieure (10), et des fibres (9) destinées au transport de la lumière sont disposées entre la paroi extérieure (10) et le boîtier étanche aux fluides (4), **caractérisé en ce que** le capteur (6) et/ou la ligne de signal (7) sont incorporés entre les fibres (9) au moins par endroits.

8. Endoscope (1) selon l'une quelconque des revendications précédentes, **caractérisé en ce que** le point de couplage (13) présente un connecteur ou un logement pour une connexion à la ligne de signal (7) afin de transmettre des signaux électriques ou optiques.

9. Endoscope (1) selon l'une quelconque des revendications précédentes, **caractérisé en ce que** le point de couplage (13, 33) est réalisé de manière étanche aux fluides.

10. Endoscope (1) selon l'une quelconque des revendications précédentes, **caractérisé en ce que** l'unité d'évaluation (12) évalue les signaux reçus du capteur (6).

11. Endoscope (1) selon la revendication 10, **caractérisé en ce que** l'endoscope (1) présente une ligne (15, 16) qui communique avec l'unité d'évaluation (12) et est adaptée pour conduire des signaux ou des données avec le résultat de l'évaluation de l'unité d'évaluation (12) à un moniteur (42) ou à un appareil médical (43) disposé à l'extérieur de l'endoscope (1).

12. Endoscope (1) selon la revendication 10 ou 11, **caractérisé en ce que** l'endoscope (1) présente un capteur (19) qui communique avec l'unité d'évaluation (12) et est adapté pour émettre des signaux ou des données avec le résultat de l'évaluation de l'unité d'évaluation (12) sans fil à un moniteur (42) ou à un appareil médical (43) situé à l'extérieur de l'endoscope (1).
